# FASCICULE DE BREVET EUROPEEN

(11) **EP 0 669 915 B1**
(45) Date de publication et mention de la délivrance du brevet: **18.06.1997**
(21) Numéro de dépôt: 94900207.5
(22) Date de dépôt: 18.11.1993
(51) Int. Cl.: C07D 263/04

(54) **PROCEDE DE PREPARATION D'UN ACIDE OXAZOLIDINE-1,3 CARBOXYLIQUE-5**
VERFAHREN ZUR HERSTELLUNG EINER 1,3-OXAZOLIDIN-5-CARBONSÄURE
METHOD FOR THE PREPARATION OF A 1,3-OXAZOLIDINE-5-CARBOXYLIC ACID

(30) Priorité: 20.11.1992 FR 9213939
(43) Date de publication de la demande: 06.09.1995
(73) Titulaire: RHONE-POULENC RORER S.A., 92160 Antony (FR)
(72) Inventeur: DENIS, Jean-Noel, Le Pinet, F-38410 Uriage (FR); GREENE, Andrew, F-38410 Uriage (FR); KANAZAWA, Alice, F-38000 Grenoble (FR)
(74) Mandataire: Pilard, Jacques
(86) Numéro de dépôt international: FR9301133
(87) Numéro de publication internationale: WO9412482

(56) Documents cités:
- WO-A-92/09589

## Description

La présente invention concerne un procédé de préparation d'un acide oxazolidine-1,3 carboxylique-5 de formule générale : 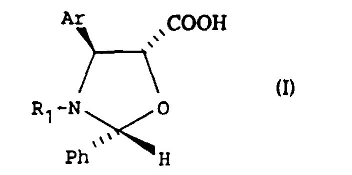 dans laquelle
Ar représente un radical phényle ou α- ou β-naphtyle éventuellement substitué par un ou plusieurs atomes ou radicaux, identiques ou différents, choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyles, mercapto, formyle, acyles, acylamino, amylamino, alcoxycarbonylamino, amino, alkylamino, diflkylamino, carboxy, alcoxycarbonyles, carbamoyle, dialcoylcarbamoyles, cyano et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou α- ou β-naphtyles,
R₁ représente un radical benzoyle ou radical R₂-O-CO- dans lequel R₂ représente
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazmyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ph représente un radical phényle substitué par un ou plusieurs radicaux alcoxy contenant 1 à 4 atomes de carbone.

Selon la présente invention, un acide de formule générale (I) peut être obtenu par cyclisation d'un produit de formule générale: 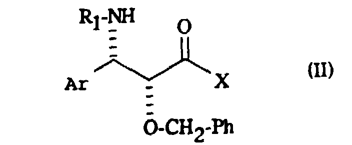 dans laquelle Ar, R₁ et Ph sont définis comme précédemment et X représente un reste de L(+)-2,10-camphorsultame de formule 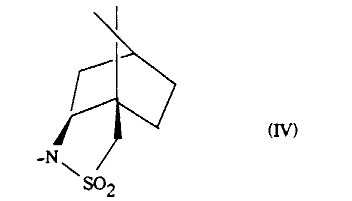 ou un reste -O-R dans lequel R représente un radical alcoyle contenant 1 à 4 atomes de carbone éventuellement substitué par un radical phényle, suivie de l'hydrolyse ou de la saponification du produit obtenu de formule générale: 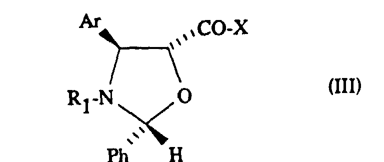 dans laquelle Ar, R₁, Ph et X sont définis comme précédemment.

La cyclisation est effectuée en opérant, de préférence en milieu anhydre, dans un solvant organique choisi parmi les éthers, les esters, les cétones, les nitriles, les hydrocarbures aliphatiques éventuellement halogénés et les hydrocarbures aromatiques éventuellement halogénés en présence d'un agent d'oxydation tel que la dichlorodicyanobenzoquinone à une température comprise entre 0°C et la température d'ébullition du mélange réactionnel. De préférence, on opère dans un hydrocarbure aliphatique halogéné à une température voisine de 20°C.

Le produit de formule générale (III) dans laquelle X représente un reste L(+)-2,10-camphorsultame de formule : 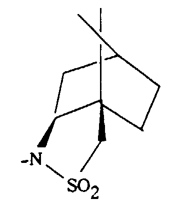 est hydrolysé en acide de formule générale (I) au moyen d'une base minérale telle que la soude, la potasse ou la lithine en milieu aqueux ou hydro-organique. Il est particulièrement avantageux d'opérer dans un mélange tétrahydrofurane-eau en présence d'eau oxygénée. La température de réaction est généralement comprise entre -10 et 20°C et de préférence voisine de 0°C.

Le produit de formule générale (III) dans laquelle X représente un reste -O-R est saponifié en acide de formule générale (I) au moyen d'une base minérale tel qu'un hydroxyde de métal alcalin (lithium, potassium, sodium), un carbonate ou bicarbonate de métal alcalin (bicarbonate de sodium, carbonate ou bicarbonate de potassium) en milieu hydro-alcoolique tel qu'un mélange méthanol-eau à une température comprise entre 10 et 40°C, de préférence voisine de 20°C.

Le produit de formule générale (II) dans laquelle X représente un reste de formule (IV) peut être obtenu par action d'une N-carbonylarylimine de formule générale :

Ar-CH=N-R₁ (V)

dans laquelle Ar et R₁ sont définis comme précédemment, sur l'anion d'un amide optiquement actif d'un acide hydroxyacétique protégé de formule générale :

Ph-CH₂-O-CO-X (VI)

dans laquelle Ph et X sont définis comme précédemment.

Généralement, on fait réagir la N-carbonylarylimine de formule générale (V), éventuellement préparée in situ, sur l'amide de l'acide hydroxyacétique préalablement anionisé de formule générale (VI) au moyen d'un amidure de métal alcalin. Parmi les amidures appropriés peuvent être cités le bis(triméthylsilyl)amidure de sodium (NHMDS), de lithium (LHMDS) ou de potassium (KHMDS), le diisopropylamidure de lithium (LDA), le diéthylamidure de lithium (LDEA), le dicyclohexylamidure de lithium (LDCHA) et (CH₃)₃SiN(R"')Li (R"' = alcoyle, cycloalcoyle, aryle). Peut également être cité le tBuLi. D'un intérêt tout particulier est le bis(triméthylsilyl) amidure de lithium qui permet d'obtenir un rendement élevé et une excellente sélectivité.

La N-carbonylarylimine de formule générale (V) peut être obtenue par action d'un halogénure de benzoyle éventuellement substitué ou d'un dérivé réactif de formule générale :

R₂-O-CO-Y (VII)

dans laquelle R₂ est défini comme précédemment et Y représente un atome d'halogène (fluor, chlore) ou un reste -O-R₂ ou -O-CO-OR₂ sur un produit de formule générale :

Ar-CH=N-Z (VIII)

dans laquelle Ar est défini comme précédemment et Z représente un groupement réactif tel qu'un radical trialkylsilyle comme le radical triméthylsilyle.

Généralement, l'action de l'halogénure de benzoyle éventuellement substitué ou des produits de formule générale (VII) sur le produit de formule générale (VIII) est effectuée par chauffage dans un solvant organique tel qu'un ester comme l'acétate d'éthyle ou un hydrocarbure aliphatique halogéné comme le dichlorométhane ou le chloroforme ou un hydrocarbure aromatique comme le toluène ou le benzène.

L'imine de formule générale (VIII) peut être obtenue à partir de l'aldéhyde de formule générale :

Ar-CHO (IX)

dans laquelle Ar est défini comme précédemment, selon les méthodes connues. Par exemple, un produit de formule générale (VIII) dans laquelle Z représente un radical triméthylsilyle peut être obtenu selon DJ. Hart et coll., J. Org. Chem., 48, 289 (1983) par action du bis(triméthylsilyl)amidure de lithium (LHMDS), éventuellement préparé in situ par action du butyllithium sur la bis(triméthylsilyl)amine sur l'aldéhyde correspondant de formule générale (IX).

L'imine de formule générale (V) peut aussi être préparée à partir d'un produit de formule générale : 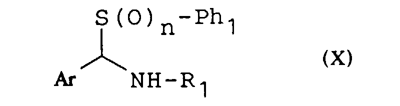 dans laquelle Ar et R₁ sont définis comme précédemment, Ph₁ représente un radical phényle éventuellement substitué, par exemple, par un radical méthyle et n est égal à 0 ou 2.

L'imine de formule générale (V) peut être préparée in situ par action d'une base forte telle qu'un amidure comme le bis(triméthylsilyl)amidune de lithium sur un produit de formule générale (X) dans laquelle n est égal à zéro.

L'imine de formule générale (V) peut être préparée par action d'une base telle que le carbonate de sodium ou de potassium dans un solvant organique choisi parmi les éthers tels que le tétahydrofurane et les hydrocarbures aromatiques tels que le benzène ou le toluène à une température comprise entre 50 et 100°C.

Le produit de formule générale (X) dans laquelle n est égal à 2 peut être préparé par action d'un sulfinate alcalin tel que le phénylsulfinate de sodium ou de potassium sur un mélange d'un aldéhyde de formule générale (IX) dans laquelle Ar est défini comme précédemment et d'un amide de formule générale :

H₂N-R₁ (XI)

dans laquelle R₁ est défini comme précédemment, en opérant dans un milieu hydro-organique tel qu'un mélange d'eau et d'un alcool aliphatique contenant 1 à 3 atomes de carbone (méthanol, éthanol) en présence d'un acide tel que l'acide formique.

L'amide optiquement active de formule générale (VI) peut être obtenue par action d'un dérivé activé d'un acide hydroxyacétique protégé de formule générale :

Ph-CH₂-O-CH₂-COOH (XII)

dans laquelle Ph est défini comme précédemment, éventuellement sous forme de sel, d'halogénure ou d'anhydride sur la base nucléophile correspondante éventuellement anionisée.

Le produit de formule générale (XII) peut être obtenu par action d'un alcoolate métallique de formule générale :

Ph-CH₂-OM (XIII)

dans laquelle Ph est défini comme précédemment et M représente un atome de métal alcalin choisi parmi le sodium, le potassium et le lithium, sur un acide de formule générale :

Hal-CH₂-COOH (XIV)

éventuellement sous forme de sel de métal alcalin, dans laquelle Hal représente un atome d'halogène choisi de préférence parmi les atomes de chlore et de brome.

Généralement la réaction s'effectue dans un solvant organique choisi parmi les éthers comme le tétrahydrofurane, les amides comme le diméthylformamide et les hydrocarbures aromatiques comme le toluène et leur mélange à une température comprise entre 20°C et la température de reflux du mélange réactionnel.

Le produit de formule générale (XII) sous forme d'halogénure peut être obtenu par action du chlorure de thionyle ou du chlorure d'oxalyle sur l'acide correspondant en opérant dans un solvant organique inerte tel qu'un hydrocarbure aliphatique comme l'hexane ou un hydrocarbure aromatique comme le benzène ou le toluène à une température voisine de 20°C.

Le produit de formule générale (II) dans laquelle X représente un reste -O-R peut être obtenu, éventuellement in situ, par action d'un imidate de formule générale : 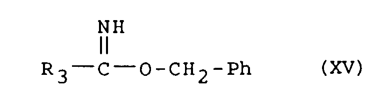 dans laquelle R₃ représente un radical alcoyle contenant 1 à 3 atomes de carbone éventuellement substitué par un ou plusieurs atomes d'halogène, de préférence un radical trichlorométhyle et Ph est défini comme précédemment, sur un ester de formule générale : 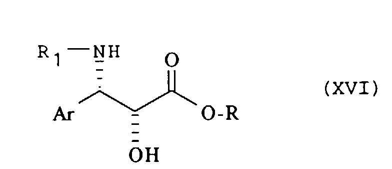 dans laquelle Ar, R₁ et R sont définis comme précédemment.

Généralement, la réaction s'effectue dans un solvant organique ou un mélange de solvants organiques choisi parmi les hydrocarbures cycloaliphatiques et les hydrocarbures aliphatiques éventuellement halogénés à une température comprise entre 0 et 50°C, de préférence voisine de 20°C.

L'ester de formule générale (XVI) peut être obtenu par acylation d'un dérivé de la phénylisosérine de formule générale : 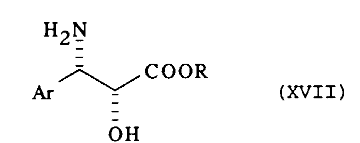 dans laquelle Ar et R sont définis comme précédemment au moyen de chlorure de benzoyle éventuellement substitué ou d'un dérivé réactif de formule générale (VII) en opérant dans un solvant organique, tel qu'un ester aliphatique comme l'acétate d'éthyle ou un hydrocarbure aliphatique halogéné comme le dichlorométhane ou éventuellement dans l'eau, en présence d'une base minérale telle que le bicarbonate de sodium ou organique telle que la triéthylamine.

Généralement la réaction est effectuée à une température comprise entre 0 et 50°C, de préférence voisine de 20°C.

Le produit de formule générale (XVII) peut être obtenu dans les conditions décrites dans la demande internationale PCT WO 92/09589.

Le produit de formule générale (II) dans laquelle X représente un reste -O-R peut aussi être obtenu à partir d'un produit de formule générale (II) dans laquelle X représente un reste de formule (IV). A cet effet, le produit de formule générale (II) dans laquelle X représente un reste de formule (IV) est hydrolysé dans les conditions décrites précédemment pour l'hydrolyse d'un produit de formule générale (III) pour fournir l'acide de formule générale : 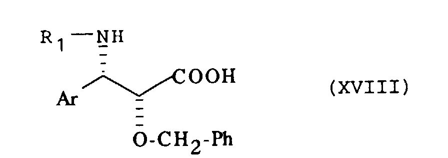 dans laquelle Ar, R₁ et Ph sont définis comme précédemment, qui est estérifié, selon les méthodes connues, pour donner le produit de formule générale (II) dans laquelle X représente un reste -O-R.

Selon la présente invention, l'acide de formule générale (I) peut aussi être obtenu par action d'un éther de formule générale :

Ph-CH₂-O-R₃ (XIX)

dans laquelle Ph est défini comme précédemment et R₃ représente un radical alcoyle contenant 1 à 4 atomes de carbone ou un radical phényle, sur un dérivé de la phénylisosérine de formule générale (XVI) sous forme 2R,3S en opérant en milieu anhydre dans un solvant organique choisi parmi les éthers, les esters, les cétones, les nitriles, les hydrocarbures aliphatiques éventuellement halogénés et les hydrocarbures aromatiques éventuellement halogénés en présence d'un agent d'oxydation tel que la dichlorodicyanobenzoquinone à une température comprise entre 0°C et la température d'ébullition du mélange réactionnel, suivie de la saponification en milieu basique de l'ester de formule générale (II) ainsi obtenu.

Le procédé selon l'invention permet d'obtenir l'acide de formule générale (I) pratiquement exempt de son épimère de formule générale : 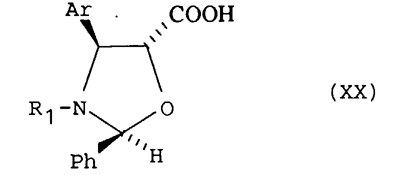 dans laquelle Ar, R₁ et Ph sont définis comme précédemment.

Il en résulte que l'acide de formule générale (I) permet de préparer les dérivés du taxane de formule générale : 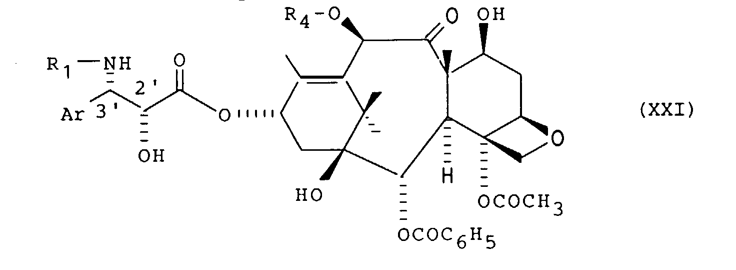 dans laquelle Ar et R₁ sont définis comme précédemment et R₄ représente un atome d'hydrogène ou un radical acétyle pratiquement exempt de l'épimère en 2'.

Les dérivés du taxane de formule générale (XXI) peuvent être obtenus par :
- condensation d'un acide de formule générale (I), ou d'un dérivé de cet acide, sur la baccatine III ou la désacétyl-10 baccatine III de formule générale :' 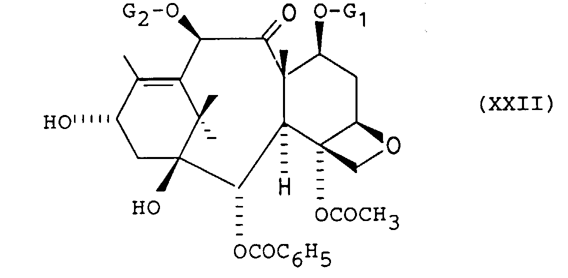 dans laquelle G₁ représente un groupement protecteur de la fonction hydroxy et G₂ représente un radical acétyle ou un groupement protecteur de la fonction hydroxy, pour obtenir un produit de formule générale : 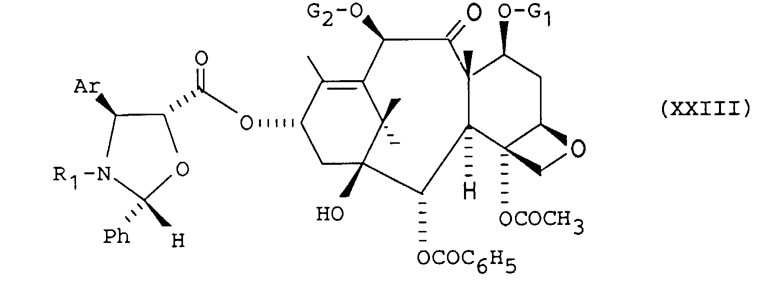 dans laquelle Ar, R₁, Ph, G₁ et G₂ sont définis comme précédemment,
- déprotection de la chaîne latérale et éventuellement des fonctions hydroxy protégées par G₁ et G₂ pour obtenir un produit de formule générale : 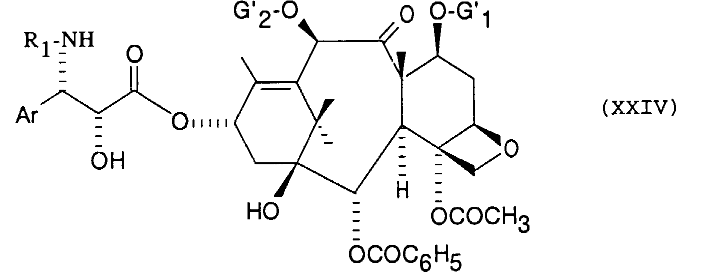 dans laquelle Ar et R₁ sont définis comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy, puis
- éventuellement remplacement des groupements protecteurs G'₁ et éventuellement G'₂ du produit de formule générale (XXIV) par des atomes d'hydrogène pour obtenir un produit de formule générale (XXI).

L'estérification du produit de formule générale (XXII) est effectuée au moyen d'un acide de formule générale (I) éventuellement sous forme d'anhydride ou sous forme d'halogénure ou d'anhvdride mixte.

L'estérification au moyen d'un acide de formule générale (I) peut être effectuée en présence d'un agent de condensation tel qu'un carbodiimide comme le dicyclohexylcarbodiimide ou un carbonate réactif comme le dipyridyl-2 carbonate et d'un agent d'activation tel qu'une aminopyridine comme la diméthylamino-4 pyridine ou la pyrrolidino-4 pyridine en opérant dans un solvant organique choisi parmi les éthers tels que le tétrahydrofurane, l'éther diisopropylique, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro1,2 éthane ou les hydrocarbures aromatiques tels que le benzène, le toluène, les xylènes, l'éthylbenzène, l'isopropylbenzène ou le chlorobenzène à une température comprise entre -10 et 90°C. Il est particulièrement avantageux d'effectuer l'estérification en opérant dans un solvant aromatique à une température voisine de 20°C.

L'estérification peut aussi être réalisée en utilisant l'acide de formule générale (I) sous forme d'anhydride de formule : 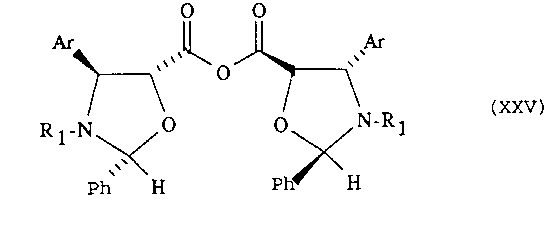 dans laquelle Ar, R₁ et Ph sont définis comme précédemment, en présence d'un agent d'activation tel qu'une aminopyridine comme la diméthylamino-4 pyridine en opérant dans un solvant organique choisi parmi les éthers tels que le tétrahydrofurane, l'éther diisopropylique, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane ou les hydrocarbures aromatiques tels que le benzène, le toluène, les xylènes, l'éthylbenzène, l'isopropylbenzène ou le chlorobenzène à une température comprise entre 0 et 90°C.

L'estérification peut aussi être réalisée en utilisant l'acide de formule générale (I) sous forme d'halogénure ou sous forme d'anhydride mixte de formule générale : 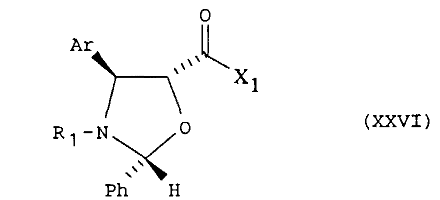 dans laquelle Ar, R₁ et Ph sont définis comme précédemment et X₁ représente un atome d'halogène ou un radical acyloxy ou aroyloxy, éventuellement préparé in situ, en présence d'une base qui est de préférence une base organique azotée telle qu'une amine aliphatique tertiaire comme la triéthylamine, la pyridine, une aminopyridine comme la diméthylamino-4 pyridine ou la pyrrolidino-4 pyridine en opérant dans un solvant organique inerte choisi parmi les éthers tels que le tétrahydrofurane, l'éther diisopropylique, le méthyl t.butyléther ou le dioxanne, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenzène, l'isopropylbenzène ou le chlorobenzène à une température comprise entre 10 et 80°C, de préférence voisine de 20°C.

De préférence, on utilise un dérivé activé de formule générale (XXVI) dans laquelle X₁ représente un atome d'halogène ou un radical acyloxy contenant 1 à 5 atomes de carbone ou aroyloxy dans lequel la partie aryle est un radical phényle éventuellement substitué par 1 à 5 atomes ou radicaux, identiques ou différents, choisis parmi les atomes d'halogène (chlore, brome) et les radicaux nitro, méthyle ou méthoxy.

La déprotection de la chaîne latérale peut être effectuée en présence d'un acide minéral (acide chlorhydrique, acide sulfurique) ou organique (acide acétique, acide méthanesulfonique, acide trifluorométhanesulfonique, acide p.toluène-sulfonique) utilisé seul ou en mélange, en opérant dans un solvant organique choisi parmi les alcools (méthanol, éthanol, propanol, isopropanol), les éthers (tétrahydrofurane, éther diisopropylique, méthyl t.butyléther), les esters (acétate d'éthyle, acétate d'isopropyle, acétate de n.butyle), les hydrocarbures aliphatiques (pentane, hexane, heptane), les hydrocarbures aliphatiques halogénés (dichlorométhane, dichloro-1,2 éthane), les hydrocarbures aromatiques (benzène, toluène, xylènes) et les nitriles (acétonitrile) à une température comprise entre -10 et 60°C, de préférence entre 15 et 30°C. L'acide minéral ou organique peut être utilisé en quantité catalytique ou stoechiométrique ou en excès.

La déprotection peut être également réalisée dans des conditions oxydantes en utilisant par exemple le nitrate d'ammonium et de cérium IV dans un mélange acétonitrile-eau ou la dichloro-2,3 dicyano-5,6 benzoquinone-1,4 dans le mélange dichlorométhane-eau.

La déprotection peut être également réalisée dans des conditions réductrices, par exemple par hydrogénolyse en présence d'un catalyseur.

Les groupements protecteurs G₁ et G₂ sont de préférence des radicaux trichloro-2,2,2 éthoxycarbonyle, (trichlorométhyl-2 propoxy)-2 carbonyle, benzyle, alcoxy-4 benzyle ou dialcoxy-2,4 benzyle dans lesquels les radicaux alcoxy contiennent 1 à 4 atomes de carbone ou des radicaux trialkylsilyles, dialkylarylsilyles, alkyldiarylsilyles ou triarylsilyles dans lesquels les parties alkyles contiennent 1 à 4 atomes de carbone et les parties aryles sont de préférence des radicaux phényles.

Le remplacement des groupements protecteurs G₁ et éventuellement G₂ représentant un radical silylé par des atomes d'hydrogène peut être effectué simultanément avec la déprotection de la chaîne latérale.

Le remplacement des groupements protecteurs G₁ et éventuellement G₂ représentant un radical trichloro-2,2,2 éthoxycarbonyle ou (trichlorométhyl-2 propoxy)-2 carbonyle, est effectué par le zinc, éventuellement associé à du cuivre, en présence d'acide acétique à une température comprise entre 20 et 70°C ou au moyen d'un acide minéral ou organique tel que l'acide chlorhydrique ou l'acide acétique en solution dans un alcool aliphatique contenant 1 à 3 atomes de carbone ou dans un ester aliphatique tel que l'acétate d'éthyle, l'acétate d'isopropyle ou l'acétate de n.butyle en présence de zinc éventuellement associé à du cuivre.

Les acides de formule générale (I) sont particulièrement utiles pour préparer les dérivés du taxane de formule générale (XXI) dans laquelle R₄ représente un radical acétyle, R₁ représente un radical benzoyle et Ar représenté un radical phényle (taxol) ou bien dans laquelle R₄ représente un atome d'hydrogène, R₁ représente un radical t.butoxycarbonyle et Ar représente un radical phényle (Taxotère).

Les exemples suivants illustrent la présente invention.

### EXEMPLE 1

Dans un ballon de 10 cm3 muni d'un système d'agitation magnétique, on introduit sous atmosphère d'argon, 157 mg (0,40 mmole) de L-(+)-N-(méthoxy-4 benzyloxyacétyl) 2,10-camphorsultame et 1,5 cm3 de tétrahydrofurane anhydre. La solution obtenue est refroidie à -30°C puis on ajoute, goutte à goutte, 0,44 cm3 (0,44 mmole) d'une solution 1M de bis(triméthylsilyl)amidure de lithium (LHMDS) dans le tétrahydrofurane. On laisse réagir pendant 15 minutes à -30°C, puis on ajoute, goutte à goutte, 130 mg (0,63 mmole) de N-t.butoxycarbonylbenzylimine en solution dans 1,0 cm3 de tétrahydrofurane anhydre. On laisse réagir pendant 15 minutes à -30°C puis on hydrolyse, à cette température, le mélange réactionnel par addition d'une solution aqueuse saturée de chlorure d'ammonium. On laisse la température remonter au voisinage de 20°C puis on extrait le mélange réactionnel 2 fois avec de l'éther éthylique. Les phases organiques réunies sont lavées 3 fois à l'eau puis 1 fois avec une solution aqueuse saturée de chlorure de sodium et enfin séchées sur sulfate de magnésium anhydre. Après filtration et élimination des solvants sous pression réduite, on obtient 290 mg d'un résidu huileux qui est purifié par chromatographie sur une colonne de gel de silice en éluant avec un mélange hexane-acétate d'éthyle (85-15 en volumes). On obtient ainsi, avec un rendement de 60 %, 146 mg (0,24 mmole) de L-(+)-N-[(méthoxy-4 benzyl)oxy-2 t.butoxycarbonylamino-3 phényl-3 propionyl] 2,10- camphorsultame-syn dont les caractéristiques sont les suivantes :
- point de fusion: 85-86°C
- pouvoir rotatoire : [α]_{D}²⁵ =+54,7° (c=0,93,CHCl₃)
- spectre infra-rouge (film) : bandes d'absorption caractéristiques à 3425, 2950, 2920, 2850, 1720, 1710, 1610, 1580, 1510, 1490, 1385, 1360, 1325, 1270, 1240, 1210, 1160, 1130, 1100, 1055, 1030, 1005, 980, 900, 720 et 690 cm⁻¹.
- spectre de résonance magnétique nucléaire du proton (300 MHz ; CDCl₃ ; déplacements chimiques en ppm ; constantes de couplage J en Hz) : 0,99 (s, 3H) ; 1,27 (s, 3H) ; 1,2-1,6 (m, 2H) ; 1,39 (s, 9H) ; 1,89-2,30 (m, 5H) ; 3,51 (AB_{q}, J_{AB} = 13,6 ; δ_{A}-δ_{B} = 21,7, 2H) ; 3,76 (s, 3H) ; 4,00 (t déformé, J = 6,0 et 6,5, 1H); 4,29 (AB_{q}, J_{AB} = 11,3 ; δ_{A}-δ_{B} = 123,3, 2H) ; 4,83 (s, 1H); 5,31 (d, J = 9,3, 1H); 5,57 (d, J = 9,3, 1H); 6,68-6,74 (m, 2H) ; 6,89-6,95 (m, 2H) ; 7,20-7,43 (m, 5H).
- spectre de résonance magnétique nucléaire du ¹³C (50,3 MHz ; CDCl₃ ; déplacements chimiques en ppm) : 19,91 (CH₃) ; 20,58 (CH₃) ; 26,53 (CH₂) ; 28,18 (CH₃) ; 32,74 ((CH₂) ; 37,49 ( CH₂) ; 44,43 (CH) ; 47,85 (C) ; 48,81 (C) ; 53,04 (CH₂) ; 55,09 (CH₃) ; 55,64 (CH) ; 65,00 (CH) ; 72,11 (CH₂) ; 79,19 (C) ; 80,88 (CH) ; 113,39 (CH) 126,71 (CH) ; 127,05 (CH) ; 127,99 (CH) ; 128,77 (C) ; 129,46 (CH) 139,56 (C) 154,86 (C) 159,09 (C) ; 169,94 (C).
- spectre de masse (I.C. ; NH₃ + isobutane) : 599 (MH+) ; 538 ; 499 ; 345 ; 233 ; 216 ; 206 ; 197 ; 180 ; 154 ; 150 ; 137 ; 121 ; 106.
- analyse élémentaire : (C₃₂H₄₂N₂O₇S)

| | | | |
|---|---|---|---|
| calculée | C % 64,19 | H % 7,07 | N % 4,68 |
| trouvée | 64,10 | 7,19 | 4,71 |

Dans un monocol de 10 cm3 muni d'un système d'agitation magnétique, on introduit, sous argon, 90 mg (0,15 mmole) de L-(+)-N-[(méthoxy-4 benzyl)oxy-2 t.butoxycarbonylamino-3 phényl-3 propionyl] 2,10-camphorsultame-syn et 2,25 cm3 de dichlorométhane sec. On ajoute ensuite à la solution résultante 15 grains de tamis moléculaire 4Å puis 102 mg (0,45 mmole) de dichlorodicyanobenzoquinone à l'aide de l'ampoule à solide. Le mélange réactionnel est agité pendant 14 heures à une température voisine de 20°C.

On dilue le mélange réactionnel dans 40 cm3 de dichlorométhane. La phase organique est lavée 3 fois avec 5 cm3 d'une solution aqueuse saturée de bicarbonate de sodium, 3 fois avec 5 cm3 d'eau puis 1 fois avec une solution aqueuse saturée de chlorure de sodium et enfin séchée sur sulfate de sodium anhydre. Après filtration et élimination du solvant sous pression réduite, on obtient 103 mg d'un résidu huileux qui est purifié par chromatographie sur colonne de gel de silice en éluant avec un mélange éther éthylique-dichlorométhane (1-99 en volumes). On obtient ainsi avec un rendement de 91 %, 81 mg (0,136 mmole) de L-(+)-N-2,10-camphorsultame t.butoxy-carbonyl-3 (méthoxy-4 phényl)-2 phényl-4 carbamoyl-5 oxazolidine-1,3-(2R,4S,5R) dont les caractéristiques sont les suivantes :
- point de fusion : 147-148°C.
- pouvoir rotatoire : [α]_{D}²⁵ =+52,9° (c = 0,95 ; CHCl₃)
- spectre infra-rouge (film) : bandes d'absorption caractéristiques à 2970, 1710, 1620, 1595, 1520, 1460, 1395, 1375, 1345, 1300, 1280, 1250, 1225, 1170, 1140, 1095, 1070, 1030 et 830 cm⁻¹.
- spectre de résonance magnétique nucléaire du proton (300 MHz ; CDCl₃ ; déplacements chimiques en ppm ; constantes de couplage J en Hz) : 0,85 (s, 3H) ; 0,90 (s, 3H) ; 1,01 (s, 9H) ; 1,26-1,52 (m, 2H) ; 1,84-1,87 (m, 3H) ; 2,07-2,10 (m, 2H) ; 3,33 (s, 2H) ; 3,75-3,88 (m, 1H); 3,81 (s, 1H); 5,16-5,27 (m, 2H) ; 6,28 (s, 1H); 6,89-6,92 (m, 2H) ; 7,20-7,40 (m, 5H) ; 7,50-7,53 (m, 2H).
- spectre de résonance magnétique nucléaire du ¹³C (50,3 MHz ; CDCl₃ ; déplacements chimiques en ppm) : 19,54 (CH₃) ; 20,38 (CH₃) ; 26,07 (CH₂) ; 27,57 (CH₃) ; 32,38 ( CH₂) ; 37,92 (CH₂) ; 44,42 (CH) ; 47,45 (C) ; 48,36 (C) ; 52,51 (CH₂); 55,06 (CH₃) ; 64,73 (CH) ; 65,31 (CH) ; 80,19 (C) ; 82,13 (CH) ; 92,41 (CH) ; 113,27 (CH) ; 126,58 (CH) ; 127,87 (CH) ; 128,38 (CH) ; 129,02 (CH) ; 130,79 (C) ; 138,20 (C) 151,33 (C) ; 159,94 (C) ; 167,86 (C).
- analyse élémentaire (C₃₂H₄₀N₂O₇S)

| | | | |
|---|---|---|---|
| calculée | C % 64,41 | H % 6,76 | N % 4,69 |
| trouvée | 64,15 | 6,85 | 4,80 |

Dans un monocol de 10 cm3 muni d'un système d'agitation magnétique, on introduit, sous argon, 27 mg (0,045 mmole) d'amide obtenu ci-dessus et 0,7 cm3 d'un mélange tétrahydrofurane-eau (4-1 en volumes). On refroidit à 0°C puis on ajoute 37 µl (0,36 mmole) d'eau oxygénée 30 % en volumes et 7,9 mg (0,188 mmole) de lithine hydratée (LiOH, H₂O). On laisse réagir pendant 30 minutes à 0°C puis on agite pendant 15 heures à 20°C.

La réaction terminée, on ajoute 10 cm3 de dichlorométhane, 10 cm3 d'eau puis 57 mg (0,45 mmole) de sulfite de sodium en poudre. On agite vigoureusement les deux phases. On les sépare par décantation puis on lave la phase aqueuse basique 3 fois avec 10 cm3 de dichlorométhane. Cette phase aqueuse est refroidie à 0°C et, sous vive agitation et en présence de 20 cm3 de dichlorométhane, elle est acidifiée avec une solution aqueuse d'acide chlorhydrique 1M jusqu'à un pH de 1-2. Elle est ensuite extraite 6 fois avec 15 cm3 de dichlorométhane. Les phases organiques réunies sont lavées 3 fois avec 5 cm3 d'eau et 1 fois par 5 cm3 d'une solution aqueuse saturée de chlorure de sodium puis séchées sur sulfate de magnésium anhydre. Après filtration et élimination des solvants sous pression réduite, on obtient, avec un rendement de 78 %, 14 mg (0,0351 mmole) d'acide t.butoxycarbonyl-3 (méthoxy-4 phényl)-2 phényl-4 oxazolidine-1,3 carboxylique-5-(2R,4S,5R) dont la pureté diastéréoisomérique en C2 est supérieure à 97 % et dont les caractéristiques sont les suivantes :
- point de fusion : 140-141°C
- spectre infra-rouge (film) : bandes d'absorption caractéristiques à 3700-2300, 2985, 2925, 1770, 1710, 1615, 1595, 1520, 1410, 1370, 1250, 1175, 1090, 1035, 920, 830, 730 et 700 cm⁻¹.
- spectre de résonance magnétique nucléaire du proton (200 MHz ; CDCl₃ ; déplacements chimiques en ppm : constantes de couplage J en Hz) : 1,06 (s, 9H) ; 3,81 (s, 3H) ; 4,61 (d, J = 4,2, 1H); 5,40 (d déformé, J = 4,7, 1H); 5,54 (s très large, 1H); 6,38 (s, 1H); 6,89-6,95 (m, 2H) ; 7,26-7,42 (m, 7H).
- spectre de résonance magnétique nucléaire du ¹³C (75,5 MHz ; CDCl₃ ; déplacements chimiques en ppm) : 27,79 (CH₃) ; 55,28 (CH₃) ; 63,67 (CH) ; 81,04 (C) ; 82,66 (CH) ; 92,39 (CH) ; 113,94 (CH) ; 126,33 (CH) ; 128,08 (CH) ; 128,37 (CH) ; 128,82 (CH) ; 130,51 (C) 140,42 (C) ; 151,71 (C) ; 160,43 (C) 172,67 (C).
- spectre de masse (I.C. ; NH₃ + isobutane) : 417 (MH⁺ + NH₃) ; 400 (MH⁺) ; 361 ; 356 ; 344 ; 317 ; 300 ; 264 ; 256 ; 236 ; 213 ; 199 ; 180 ; 154 ; 137 ; 124 ; 110.
- analyse élémentaire (C₂₂H₂₅NO₆)

| | | | |
|---|---|---|---|
| calculée | C % 66,15 | H % 6,31 | N % 3,51 |
| trouvée | 66,01 | 6,35 | 3,56 |

Le L-(+)-N-(méthoxy-4 benzyloxyacétyl) 2,10-camphorsultame peut être préparé de la manière suivante :

Dans un monocol de 500 cm3 muni d'un système d'agitation magnétique, on introduit 3,16 g (79 mmoles) d'hydrure de sodium 60 % en suspension dans l'huile et 140 cm3 de tétrahydrofurane anhydre. On refroidit la suspension résultante à 0°C puis on lui additionne 3,036 g (22 mmoles) de p-méthoxybenzylalcool en solution dans 70 cm3 de tétrahydrofurane anhydre. On laisse réagir le mélange réactionnel pendant 30 minutes à 0°C puis on introduit, par petites portions, 4,6 g (33 mmoles) d'acide bromoacétique. On chauffe le mélange réactionnel au reflux du solvant pendant 18 heures. On laisse revenir à une température voisine de 20°C, puis on élimine le tétrahydrofurane sous pression réduite. On met le résidu obtenu en solution dans de l'éther éthylique et on verse la solution dans de l'eau refroidie à 0°C. On acidifie le milieu résultant avec une solution aqueuse d'acide chlorhydrique 10 %. On sépare les deux phases obtenues. La phase organique est lavée 2 fois avec de l'eau puis 1 fois avec une solution aqueuse saturée de chlorure de sodium, puis elle est séchée sur sulfate de magnésium anhydre. Après filtration et élimination du solvant sous pression réduite, on obtient 4,9 g d'un résidu huileux qui est purifié par chromatographie sur colonne de gel de silice en éluant avec un mélange hexane-acétate d'éthyle (50-50 en volumes). On obtient 4,3 g (22 mmoles) d'acide p.méthoxybenzyloxyacétique dont les caractéristiques sont les suivantes :
- point de fusion : 54-55°C.
- spectre infra-rouge (film) : bandes d'absorption caractéristiques à 3600-2300, 2940, 1730, 1615, 1590, 1520, 1305, 1250, 1180, 1110, 1035 et 820 cm⁻¹.
- spectre de résonance magnétique nucléaire du proton (200 MHz ; CDCl₃ ; déplacements chimiques en ppm ; constantes de couplage J en Hz) : 3,81 (s, 3H) ; 4,10 (s, 2H) ; 4,58 (s, 2H) ; 6,90 (d, J = 8,6, 2H) ; 7,28 (d, J = 8,6, 2H).
- spectre de résonance magnétique nucléaire du ¹³C (50,3 MHz ; CDCl₃ ; déplacements chimiques en ppm) : 55,14 (CH₃) ; 66,06 (CH₂) ; 72,92 (CH₂) ; 113,86 (CH) ; 128,56 (C) ; 129,40 (C) ; 159,48 (C) ; 175,66 (C).

Dans un monocol de 500 cm3 muni d'un système d'agitation magnétique, on introduit, sous argon, 1,0 g (5,1 mmoles) d'acide obtenu ci-dessus, 210 cm3 d'hexane sec et 0,4 cm3 (380 mg, 5,2 mmoles) de N,N-diméthylformamide. On ajoute ensuite à la solution résultante 2,1 cm3 (3,055 g, 24,07 mmoles) de chlorure d'oxalyle fraîchement distillé. On laisse réagir le mélange réactionnel 1 heure à une température voisine de 20°C. On sépare la phase organique d'un résidu huileux insoluble qui se trouve au fond du ballon et on élimine sous pression réduite l'excès de chlorure d'oxalyle et l'hexane. Le résidu résultant est purifié par distillation sous pression réduite (0,01 mm de mercure ; 0,013 kPa). On obtient ainsi, avec un rendement de 79 %, 863 mg (4,02 mmoles) du chlorure de l'acide p.méthoxybenzyloxyacétique dont les caractéristiques sont les suivantes :
- spectre infra-rouge (film) : bandes d'absorption caractéristiques à 2970, 2930, 2875, 1810, 1620, 1590, 1520, 1470, 1420, 1390, 1305, 1260, 1190, 1180, 1130, 1040, 940, 820, 770 et 750 cm⁻¹.

Dans un ballon de 15 cm3 muni d'un système d'agitation magnétique, on met 611 mg (2,84 mmoles) de L-(+)-2,10-camphorsultame en solution dans 6 cm3 de toluène anhydre. On refroidit la solution à 0°C puis on ajoute 168 mg (4,2 mmoles) d'hydrure de sodium 60 % en suspension dans de l'huile. On laisse réagir le mélange réactionnel pendant 30 minutes à une température voisine de 20°C. On refroidit à nouveau à 0°C puis on ajoute 763 mg (3,56 mmoles) de chlorure d'acide p.méthoxybenzylacétique. On laisse remonter à une température voisine de 20°C puis on laisse réagir pendant 15 heures. La réaction terminée, on dilue le mélange réactionnel résultant par addition de dichlorométhane puis on ajoute de l'eau très lentement. On lave la phase organique 1 fois avec de l'eau puis 1 fois avec une solution aqueuse saturée de chlorure de sodium puis on la sèche sur sulfate de magnésium anhydre. Après filtration et évaporation du dichlorométhane sous pression réduite, on obtient 1,34 g d'un résidu huileux qui est purifié par chromatographie sur colonne de gel de silice en éluant avec un mélange hexane-acétate d'éthyle (80-20 en volumes). On obtient, avec 90 % de rendement, 1,01 g (2,57 mmoles) de L-(+)-N-[(méthoxy-4 benzyloxyacétyl) 2,10-camphorsultame dont les caractéristiques sont les suivantes :
- pouvoir rotatoire : [α]_{D}²⁵ = +87,1° (c = 3,25 ; CHCl₃)
- spectre infra-rouge (film) : bandes d'absorption caractéristiques à 2970, 1715, 1620, 1590, 1520, 1470, 1415, 1400, 1380, 1340, 1280, 1250, 1240, 1220, 1170, 1135, 1115, 1060, 1035, 985, 820 et 780 cm⁻¹.
- spectre de résonance magnétique nucléaire du proton (200 MHz ; CDCl₃ ; déplacements chimiques en ppm ; constantes de couplage J en Hz) : 0,96 (s, 3H) ; 1,12 (s, 3H) ; 1,2-1,6 (m, 2H) ; 1,8-2,3 (m, 5H) ; 3,3-3,6 (m, 2H) ; 3,79 (s, 3H) ; 3,8-4,0 (m, 1H); 4,3-4,7 (m, 4H) ; 6,87 (d, J = 8,6, 2H) ; 7,30 (d, J = 8,6, 2H).
- spectre de résonance magnétique nucléaire du ¹³C (50,3 MHz ; CDCl₃ ; déplacements chimiques en ppm) : 19,62 (CH₃) ; 20,53 (CH₃) ; 26,18 (CH₂) ; 32,51 (CH₂) ; 37,98 (CH₂) ; 44,37 (CH) ; 47,57 (C) ; 49,02 (C) ; 52,41 (CH₂) ; 55,02 (CH₃) ; 64,73 (CH) ; 68,03 (CH₂) ; 73,00 (CH₂) ; 113,60 (CH) ; 129,01 (C) ; 129,60 (C) ; 159,21 (C) 168,82 (C).
- analyse élémentaire (C₂₀H₂₇NO₅S)

| | | | |
|---|---|---|---|
| calculée | C % 61,07 | H % 6,87 | N % 3,56 |
| trouvée | 61,23 | 7,05 | 3,62 |

### EXEMPLE 2

Dans un ballon de 10 cm3 muni d'un système d'agitation magnétique, on met, sous atmosphère d'argon, 5 mg (0,125 mmole) d'hydrure de sodium, à 60 % dans l'huile minérale, en suspension dans 2,5 cm3 d'éther anhydre. On ajoute ensuite 156 µl (172,8 mg, 1,251 mmole) d'alcool méthoxy-4 benzylique pur. On agite (dégagement gazeux) la solution homogène résultante pendant 30 minutes à une température voisine de 20°C puis on la refroidit à 0°C. On ajoute ensuite 125 µl (180 mg, 1,20 mmole) de trichloroacétonitrile. On laisse réagir pendant 4 heures en laissant remonter lentement la température au voisinage de 20°C. On concentre le mélange réactionnel sous pression réduite jusqu'à obtenir une huile jaune orange qui est remise en solution dans 1,7 cm3 d'hexane contenant 5,5 µl de méthanol sec. On filtre la suspension sur célite sous pression réduite. On lave les solides obtenus 1 fois avec 5 cm3 d'hexane puis on élimine les solvants sous pression réduite.L'huile jaune obtenue (trichloroacétimidate de méthoxy-4 benzyle) est mise en solution dans 2 cm3 de cyclohexane. On additionne ensuite 240 mg (0,81 mmole) de t.butoxycarbonyl-amino-3 phényl-3 hydroxy-2 propionate-(2R,3S) de méthyle, 1,0 cm3 de dichlorométhane sec et 4 µl d'éthérate de trifluorure de bore. On laisse réagir à une température voisine de 20°C pendant 13 heures. On filtre le mélange réactionnel sur célite et on lave les solides 3 fois avec 10 cm3 d'un mélange dichlorométhane-cyclohexane (1-2 en volumes). On lave la phase organique résultante 2 fois avec 5 cm3 d'une solution aqueuse saturée en hydrogénocarbonate de sodium, 3 fois avec 5 cm3 d'eau et 1 fois avec 5 cm3 d'une solution aqueuse saturée de chlorure de sodium. On la sèche sur sulfate de magnésium anhydre. Après filtration et évaporation des solvants sous pression réduite, on purifie le résidu obtenu (547 mg) par chromatographie sur colonne de gel de silice en éluant avec un mélange hexane-acétate d'éthyle (70-30 en volumes). On obtient 282 mg d'un solide que l'on recristallise dans le mélange dichlorométhane-hexane à 0°C. Après séparation des cristaux obtenus (impuretés), la phase liquide ("eaux-mères") est évaporée sous pression réduite. On obtient, avec un rendement de 68 %, 228 mg (0,55 mmole) de t.butoxycarbonylamino-3 phényl-3 (méthoxy-4 benzyl)oxy-2 propionate-(2R,3S) de méthyle dont les caractéristiques sont les suivantes :
- point de fusion : 105-106°C (cyclohxane-dichlorométhane).
- pouvoir rotatoire [α]²⁵_{D} = +37,6° (c = 1,1, chloroforme).
- spectre infra-rouge (film) : bandes d'absorption caractéristiques à 3440, 2970, 2950, 2840, 1760, 1720, 1620, 1590, 1455, 1420, 1395, 1370, 1215, 1170, 1110, 1030, 820 et 700 cm⁻¹.
- spectre de résonance magnétique nucléaire du proton (200 MHz ; CDCl₃ ; déplacements chimiques en ppm ; constantes de couplage J en Hz) : 1,39 (s, 9H) ; 3,78 (s, 6H) ; 4,12 (d, J = 2, 1H); 4,40 (AB_{q}, J_{AB} = 11,4, δ_{A}-δ_{B} = 78,3, 2H) ; 5,20 (d déformé, J = 9, 1H); 5,60 (d déformé, J = 9, 1H); 6,68-6,76 (m, 2H) ; 6,83-6,95 (m, 2H) ; 7,18-7,36 (m, 5H).
- spectre de résonance magnétique nucléaire du ¹³C (100 MHz ; CDCl₃ ; déplacements chimiques en ppm) : 28,18 (CH₃) ; 52,17 (CH₃) ; 55,16 (CH₃) ; 55,99 (CH) ; 72,37 (CH₂); 79,62 (C) ;79,68 (CH) ; 113,58 (CH) ; 126,57 (CH) ; 127,32 (CH) ; 128,23 (CH) ; 128,58 (C) ; 129,54 (CH) ; 139,49 ; (C) ; 155,08 (C) 159,26 (C); 170,77 (C).
- spectre de masse (I.C. ; NH₃ + isobutane) : 433 (MH⁺ + NH₃) ; 416 (MH⁺) ; 377 ; 360 ; 354 ; 316 ; 206 ; 162 ; 138 ; 121 ; 106.
- analyse élémentaire (C₂₃H₂₉NO₆)

| | | | |
|---|---|---|---|
| calculée | C % 66,49 | H % 7,03 | N % 3,37 |
| trouvée | 66,27 | 7,07 | 3,31 |

Dans un ballon de 15 cm3 muni d'un système d'agitation magnétique, on introduit, sous atmosphère d'argon, 130 mg (0,31 mmole) du produit obtenu ci-dessus et 4,5 cm3 de dichlorométhane sec. On ajoute ensuite, à la solution résultante, 10 grains de tamis moléculaire 4Å puis 211 mg (0,93 mmole) de dichlorodicyanobenzoquinone à l'aide d'une ampoule à solide. Le mélange réactionnel est agité pendant 16 heures à une température voisine de 20°C. On dilue le mélange réactionnel dans 40 cm3 de dichlorométhane. On lave la phase organique 3 fois avec 5 cm3 d'une solution aqueuse saturée d'hydrogénocarbonate de sodium, 3 fois avec 5 cm3 d'eau puis 1 fois avec 5 cm3 d'une solution aqueuse saturée de chlorure de sodium et la sèche sur sulfate de sodium anhydre. Après filtration et élimination du solvant sous pression réduite, on obtient 130 mg d'un résidu qui est purifié par chromatographie sur colonne de gel de silice avec un mélange d'éther éthylique-hexane (20-80 en volumes). On obtient ainsi, avec un rendement de 66 %, 85 mg (0,206 mmole) de t.butoxycarbonyl-3 phényl-4 (méthoxy-4 phényl)-2 méthoxy-carbonyl-5 oxazolidine-1,3-(2R,4S,5R) pur dont les caractéristiques sont les suivantes :
- point de fusion : 104-105°C (dichlorométhane-hexane).
- pouvoir rotatoire : [α]_{D}²⁵ = +58,8° (c = 0,85 ; CHCl₃).
- spectre infra-rouge (film) : bandes d'absorption caractéristiques à 2970, 2950, 2920, 2840, 1765, 1740, 1710, 1620, 1590, 1520, 1395, 1385, 1375, 1255, 1175, 1140, 1030, 930 et 830 cm⁻¹.
- spectre de résonance magnétique nucléaire du proton (200 MHz ; CDCl₃ ; déplacements chimiques en ppm ; constantes de couplage J en Hz) : 1,07 (s, 9H) ; 3,59 (s, 3H) ; 3,81 (s, 3H) ; 4,57 (d, J = 4, 1H); 5,41 (s large, 1H); 6,37 (s large, 1H); 6,91 (d, J = 8,8, 2H) ; 7,28-7,40 (m, 7H).
- spectre de résonance magnétique nucléaire du ¹³C (50,3 MHz, CDCl₃) : 27,81 (CH₃) ; 52,42 (CH₃) ; 55,27 (CH₃) ; 63,30 (CH) ; 80,64 (C) ; 83,00 (CH) ; 91,95 (CH) ; 113,63 (CH) ; 126,34 (CH) ; 127,92 (CH) ; 128,32 (CH) ; 128,72 (CH) ; 130,85 (C) ; 140,74 (C) ; 151,69 (C) ; 160,10 (C) ; 170,20 (C).
- spectre de masse (i.c. ; NH₃ + isobutane) : 414 (MH⁺) ; 375 ; 358 ; 314 ; 250 ; 206.
- analyse élémentaire (C₂₃H₂₇NO₆)

| | | | |
|---|---|---|---|
| calculée | C % 66,81 | H % 6,58 | N % 3,39 |
| trouvée | 66,85 | 6,56 | 3,45 |

Dans un ballon de 15 cm3 muni d'un système d'agitation magnétique, on introduit, sous atmosphère d'argon, 42 mg (0,102 mmole) du produit pur obtenu ci-dessus et 4 cm3 de méthanol. On ajoute ensuite 2 cm3 d'eau distillée et 41,4 mg (0,3 mmole) de carbonate de potassium solide. On laisse réagir le mélange réactionnel à une température voisine de 20°C pendant 15 heures. La réaction terminée, on élimine le méthanol sous pression réduite puis on ajoute 10 cm3 d'eau. La phase aqueuse basique est extraite 3 fois par 10 cm3 de dichlorométhane. Cette phase aqueuse est ensuite refroidie à 0°C puis, sous vive agitation et en présence de 20 cm3 de dichlorométhane, on l'acidifie avec une solution d'acide chlorhydrique 1M jusqu'à un pH = 1-2. On extrait la phase aqueuse acide 5 fois avec 15 cm3 de dichlorométhane. Les phases organiques réunies sont lavées 3 fois par 5 cm3 d'eau, 1 fois par 5 cm3 d'une solution aqueuse saturée de chlorure de sodium puis séchées sur sulfate de magnésium anhydre. Après filtration et élimination des solvants sous pression réduite, on obtient, avec 98 % de rendement, 40 mg (0,1 mmole) d'acide t.butoxycarbonyl-3 (méthoxy-4 phényl)-2 phényl-4 oxazolidine-1,3 carboxylique-5-(2R,4S,5R) dont les caractéristiques sont identiques à celles du produit obtenu à l'exemple 1.

### EXEMPLE 3

Dans un ballon de 10 cm3, muni d'une agitation magnétique et surmonté d'un système de distillation et d'une ampoule à solide, on introduit successivement 104 mg (0,35 mmole) de t.butoxycarbonylamino-3 phényl-3 hydroxy-2 propionate-(2R,3S) de méthyle, 243 mg (1,6 mmole) de p.méthoxyphénylméthyl méthyléther, 5 cm3 d'acétonitrile fraîchement distillé sur hydrure de calcium et quelques grains de tamis moléculaire 4Å. On chauffe le mélange réactionnel au reflux puis on ajoute, en une fois, 217 mg (0,96 mmole) de dichlorodicyanobenzoquinone à l'aide de l'ampoule à solide. On agite pendant 10 minutes au reflux en éliminant le méthanol formé par distillation. On dilue le mélange réactionnel dans du dichlorométhane sec puis on filtre sur célite. La célite est rincée 4 fois avec du dichlorométhane. Les phases organiques réunies sont lavées 3 fois avec une solution aqueuse de bicarbonate de sodium à 5 % (p/v), 1 fois avec une solution aqueuse saturée de chlorure de sodium puis séchées sur sulfate de magnésium anhydre. Après filtration et élimination des solvants sous pression réduite, on obtient 331 mg d'un résidu huileux qui est purifié par chromatographie sur une colonne de gel de silice (silice imprégnée de 2,5 % (v/v) de triéthylamine) en éluant avec un mélange hexane-acétate d'éthyle (95-5 en volumes). On obtient ainsi 136 mg d'un produit "déliquescent" qui est recristallisé dans un mélange dichlorométhane-hexane. On obtient ainsi, avec un rendement de 77 %, 110 mg (0,27 mmole) de t.butoxycarbonyl-3 phényl-4 (méthoxy-4 phényl)-2 oxazolidine-1,3-(2R,4S,5R) dont les caractéristiques sont identiques à celles du produit obtenu à l'exemple 2.

### EXEMPLE 4

Dans un réacteur de 25 cm3, muni d'une agitation mécanique et d'un thermomètre, on introduit, sous atmosphère d'argon, 0,29 g de L-(+)-N-(méthoxy-4 benzyloxyacétyl)-2,10 camphorsultame et 3 cm3 de tétrahydrofuranne. On ajoute alors, à -72°C, 0,808 cm3 d'une solution 1M de bis(triméthylsilyl)amidure de lithium dans le tétrahydrofurane. Après 15 minutes d'agitation, on ajoute une solution de 0,2 g de N-benzoylbenzylimine dans 1,5 cm3 de tétrahydrofurane. On agite pendant 45 minutes à -78°C puis on hydrolyse le mélange réactionnel par addition de 0,6 cm3 d'acide chlorhydrique à 10 %. On ajoute 10 cm3 d'éther isopropylique et 2 cm3 d'eau. Le pH du mélange réactionnel est ajusté à 4 par addition d'acide chlorhydrique concentré. La phase aqueuse est séparée par décantation puis on lave la phase organique 3 fois par 2 cm3 d'eau et 1 fois par 2 cm3 d'une solution aqueuse saturée de chlorure de sodium. La phase organique est séchée sur sulfate de sodium anhydre. Après filtration et concentration sous pression réduite, on obtient 0,522 g d'une huile qui est purifiée par chromatographie sur 17 g de gel de silice en éluant avec un mélange acétate d'éthyle- heptane (30-70 en volumes). On obtient ainsi, avec un rendement de 80,8 %, 0,35 g de L-(+)-N-[(méthoxy-4 benzyl)oxy-2 benzoylamino-3 phényl-3 propionyl]-2,10 camphosultame sous forme d'un mélange syn/anti (85/15).

Le produit ainsi obtenu est traité dans les conditions décrites dans l'exemple 1 pour donner l'acide benzoyl-3 (méthoxy-4 phényl)-2 phényl-4 oxazolidine-1,3 carboxylique-5-(2R,4S,5R).

La N-benzoylbenzylimine peut être préparée de la manière suivante :

Dans un réacteur de 500 cm3 muni d'une agitation magnétique et d'un réfrigérant, on introduit une solution de 40,2 g de phénylsulfinate de sodium dans 200 cm3 d'eau. On ajoute alors un mélange de 12,35 g de benzamide et 21,22 g de benzaldéhyde. On ajoute alors 60 cm3 de méthanol de façon à obtenir une émulsion blanchâtre, puis 7,54 cm3 d'acide formique (d = 1,22). Après 41 heures 30 minutes d'agitation à une température voisine de 20°C, on chauffe pendant 3 heures à 65°C. Après filtration sur verre fritté No. 4, le produit obtenu est lavé par 2 fois 15 cm3 d'éther isopropylique puis par 2 fois 15 cm3 d'eau. Après séchage, on obtient avec un rendement de 15,6 %, 5,48 g de N-(α-phénylsulfonylbenzyl)-benzamide.

A partir des eaux-mères de filtration, on récupère 15,4 g de N-(α-phénylsulfonylbenzyl)-benzamide.

Le rendement global est voisin de 60 %.

A une solution de 1 g de la sulfone obtenue précédemment dans 50 cm3 de tétrahydrofurane chauffée à 55°C, on ajoute 0,472 g de carbonate de potassium. On chauffe à 55°C pendant 3 heures 30 minutes puis pendant 2 heures 30 minutes au reflux. Après refroidissement à une température voisine de 0°C, le précipité formé est séparé par filtration sur verre fritté No. 4. Le filtrat est concentré à sec puis repris par 5 cm3 d'éther isopropylique. Le précipité blanc qui se forme est séparé par filtration puis lavé par de l'éther isopropylique. On récupère ainsi 0,162 g de sulfone de départ.

Le filtrat, après concentration à sec, fournit 0,46 g de N-benzoylbenzylimine dont la structure est confirmée par le spectre de résonance magnétique nucléaire du proton.

## Revendications

1. Procédé de préparation d'un acide oxazolidine-1,3 carboxylique-5 de formule générale : 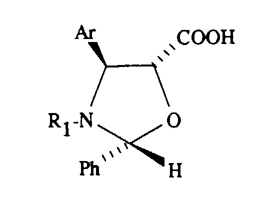 dans laquelle
Ar représente un radical phényle ou α- ou β-naphtyle éventuellement substitué par un ou plusieurs atomes ou radicaux, identiques ou différents, choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyles, mercapto, formyle, acyles, acylamino, aroylamino, alcoxycarbonylamino, amino, alkylamino, dialkylamino, carboxy, alcoxycarbonyles, carbamoyle, dialcoylcarbamoyles, cyano et trifluométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou α- ou β-naphtyles,
R₁ représente un radical benzoyle ou un radical R₂-O-CO- dans lequel R₂ représente
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclyle azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ph représente un radical phényle substitué par un ou plusieurs radicaux alcoxy contenant 1 à 4 atomes de carbone,
caractérisé en ce que l'on effectue la cyclisation d'un produit de formule générale : 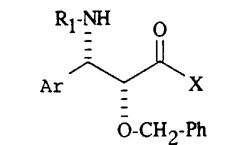 dans laquelle Ar, R₁ et Ph sont définis comme précédemment et X représente un reste de L(+)-2,10-camphorsultame de formule 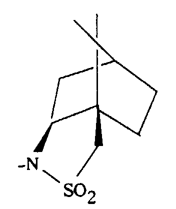 ou un reste -O-R dans lequel R représente un radical alcoyle contenant 1 à 4 atomes de carbone éventuellement substitué par un radical phényle en opérant dans un solvant organique en présence d'un agent d'oxydation à une température comprise entre 0°C et la température d'ébullition du mélange réactionnel, suivie de l'hydrolyse ou de la saponification, dans des conditions connues, du produit obtenu de formule générale : 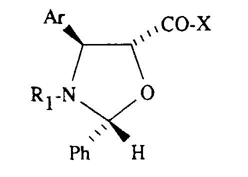 dans laquelle Ar, R₁, Ph et X sont définis comme précédemment.

2. Procédé selon la revendication 1 caractérisé en ce que le solvant est choisi parmi les éthers, les esters, les cétones, les nitriles, les hydrocarbures aliphatiques éventuellement halogénés et les hydrocarbures aromatiques.

3. Procédé selon l'une des revendications 1 ou 2 caractérisé en ce que le solvant organique est anhydre.

4. Procédé selon la revendication 1 caractérisé en ce que l'agent d'oxydation est la dichlorodicyanobenzoquinone.

5. Procédé selon l'une des revendications 1 à 4 caractérisé en ce que X est un reste de L(+)-2,10-camphorsultame de formule :

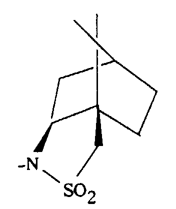

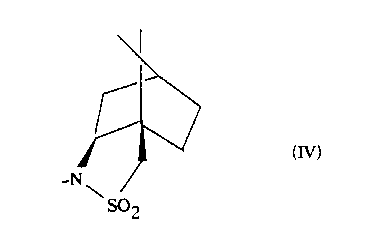

6. Procédé de préparation d'un acide oxazolidine-1,3 carboxylique-5 tel que défini dans la revendication 1 caractérisé en ce que l'on fait réagir un éther de formule générale :
Ph-CH₂-O-R₃
dans laquelle Ph est défini comme dans la revendication 1 et R₃ représente un radical alcoyle contenant 1 à 4 atomes de carbone ou un radical phényle, sur un dérivé de la phénylisosérine de formule générale : 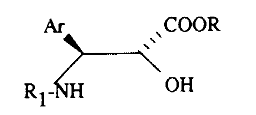 dans laquelle Ar, R₁ et R sont définis comme dans la revendication 1, en opérant dans un solvant organique en présence d'un agent d'oxydation à une température comprise entre 0°C et la température d'ébullition du mélange réactionnel, puis saponifie en milieu basique selon les méthodes connues l'ester obtenu de formule générale : 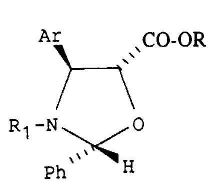 dans laquelle Ar, R₁, R et Ph sont définis comme dans la revendication 1.

7. Procédé selon la revendication 6 caractérisé en ce que le solvant est choisi parmi les éthers, les esters, les cétones, les nitriles, les hydrocarbures aliphatiques éventuellement halogénés et les hydrocarbures aromatiques éventuellement halogénés.

8. Procédé selon la revendication 7 caractérisé en ce que le solvant organique est anhydre.

9. Procédé selon la revendication 6 caractérisé en ce que l'agent d'oxydation est la dichlorodicyanobenzoquinone.

10. Procédé de préparation de taxane de formule générale 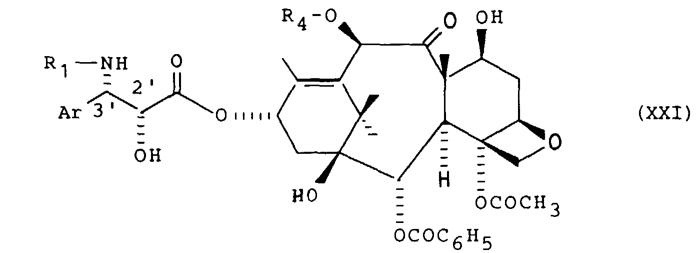 dans laquelle Ar et R₁ sont définis comme dans la revendication 1 et R₄ représente un atome d'hydrogène ou un radical acétyle caractérisé en ce que l'on effectue
- la condensation d'un acide de formule générale (I) obtenu selon la revendication 1, ou d'un dérivé de cet acide, sur la baccatine III ou la désacétyl-10 baccatine III de formule générale: 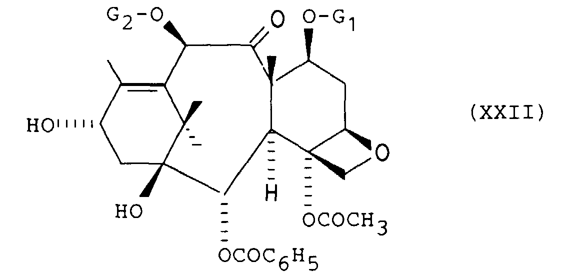 dans laquelle G₁ représente un groupement protecteur de la fonction hydroxy et G₂ représente un radical acétyle ou un groupement protecteur de la fonction hydroxy, pour obtenir un produit de formule générale : 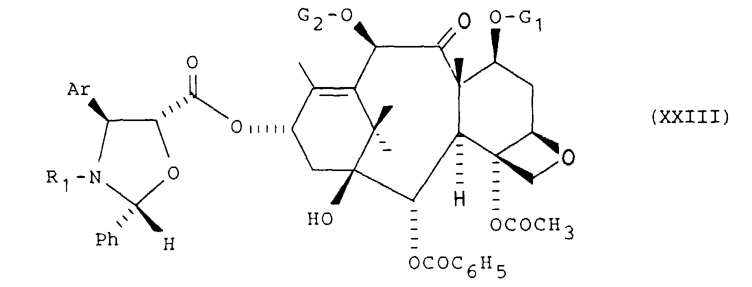 dans laquelle Ar, R₁, Ph, G₁ et G₂ sont définis comme précédemment,
- la déprotection de la chaîne latérale et éventuellement des fonctions hydroxy protégées par G₁ et G₂ pour obtenir un produit de formule générale : 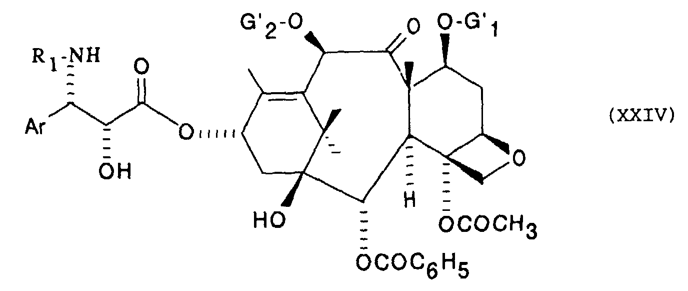 dans laquelle Ar et R₁ sont définis comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy,
- puis éventuellement le remplacement des groupements protecteurs G'₁ et éventuellement G'₂ du produit de formule générale (XXIV) par des atomes d'hydrogène pour obtenir un produit de formule générale (XXI).

11. Procédé selon la revendication 10 caractérisé en ce que la condensation est effectuée en présence d'un agent de condensation choisi parmi les carbodiimides ou les carbonates réactifs et d'un agent d'activation choisi parmi les aminopyridines.

12. Procédé selon la revendication 10 caractérisé en ce que la condensation est effectuée dans un solvant organique choisi parmi les éthers, les cétones, les esters, les nitriles, les hydrocarbures aliphatiques éventuellement halogénés, les hydrocarbures aromatiques.

13. Procédé selon la revendication 10 caractérisé en ce que le dérivé de l'acide formule (I) est un anhydride de formule générale 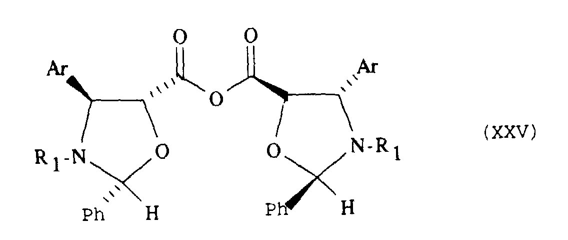 dans laquelle Ar, R₁ et Ph sont définis comme dans la revendication 1.

14. Procédé selon la revendication 10 caractérisé en ce que le dérivé de l'acide de formule (I) est un halogénure ou un anhydride mixte de formule 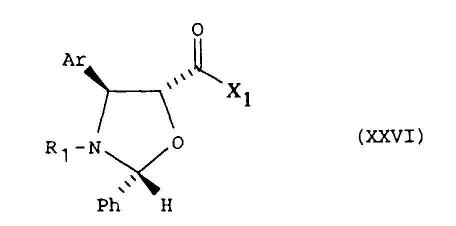 dans laquelle Ar, R₁ et Ph sont définis comme dans la revendication 1 et X₁ représente un atome d'halogène ou un radical acyloxy contenant 1 à 5 atomes de carbone ou aroyloxy dans lequel la partie aryle est un radical phényle éventuellement substitué par 1 à 5 radicaux, identiques ou différents, choisis parmi les atomes d'halogène, les radicaux nitro, méthyle ou méthoxy.

15. Procédé selon la revendication 10 caractérisé en ce que la déprotection de la chaîne latérale est effectuée en présence d'un acide minéral ou organique.

16. Procédé selon la revendication 15 caractérisé en ce que la déprotection est effectuée dans un solvant organique choisi parmi les alcools, les éthers, les esters, les hydrocarbures aliphatiques, les hydrocarbures aliphatiques halogénés, les hydrocarbures aromatiques et les nitriles.

17. Procédé selon la revendication 10 caractérisé en ce que la déprotection est effectuée dans des conditions oxydantes en utilisant le nitrate d'ammonium et de cérium IV ou la dichloro-2,3 dicyano-5,6 benzoquinone-1,4.

18. Procédé selon la revendication 10 caractérisé en ce que les groupements protecteurs G₁ et G₂ sont des radicaux trichloro-2,2,2 éthoxycarbonyle, (trichlorométhyl-2 propoxy)-2 carbonyle, benzyle, alcoxy-4 benzyle ou dialcoxy-2,4 benzyle dans lesquels les radicaux alcoxy contiennent 1 à 4 atomes de carbone ou des radicaux trialkylsilyles, dialkylarylsilyles, alkyldiarylsilyles ou triarylsilyles dans lesquels les parties alkyles contiennent 1 à 4 atomes de carbone et les parties aryles sont des radicaux phényles.

19. Procédé selon la revendication 10 caractérisé en ce que la déprotection des groupements protecteurs G₁ et éventuellement G₂ représentant un radical trichloro-2,2,2 éthoxycarbonyle ou (trichlorométhyl-2 propoxy)-2 carbonyle, est effectuée par le zinc, éventuellement associé à du cuivre.

## Patentansprüche

1. Verfahren zur Herstellung einer 1,3-Oxazolidin-5-carbonsäure der allgemeinen Formel 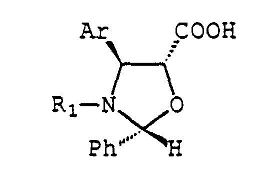 in der
Ar einen Rest Phenyl oder α- oder β-Naphthyl darstellt, gegebenenfalls substituiert durch ein oder mehrere gleiche oder verschiedene Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Arylamino, Alkoxycarbonylamino, Amino, Alkylamino, Dialkylamino, Carboxy, Alkoxycarbonyl, Carbamoyl, Dialkylcarbamoyl, Cyano und Trifluormethyl, mit der Maßgabe, daß die Alkylreste und Alkylteile der anderen Reste 1 bis 4 Kohlenstoffatome enthalten, daß die Reste Alkenyl und Alkinyl 3 bis 8 Kohlenstoffatome enthalten und daß die Arylreste Phenylreste oder α- oder β-Naphthylreste sind,
R₁ einen Rest Benzoyl oder einen Rest R₂-O-CO- darstellt, worin R₂ ist:
- ein gerader oder verzweigter Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 11 Kohlenstoffatomen, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, von dem jeder Alkylteil 1 bis 4 Kohlenstoffatome enthält, Piperidino, Morpholino, 1-Piperazinyl (gegebenenfalls substituiert in -4 durch einen Alkylrest mit 1 bis 4 Kohlenstoffatomen oder durch einen Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Carboxy oder Alkyloxycarbonyl, dessen Alkylteil 1 bis 4 Kohlenstoffatome enthält,
- oder ein Rest Phenyl, gegebenenfalls substituiert durch ein oder mehrere Atome oder Reste, ausgewählt unter den Resten Alkyl mit 1 bis 4 Kohlenstoffatomen oder Alkyloxy mit 1 bis 4 Kohlenstoffatomen,
- oder ein Rest eines gesättigten oder ungesättigten Stickstoff-Heterocyclus mit 5 oder 6 Ringgliedern und gegebenenfalls substituiert durch einen oder mehrere Alkylreste mit 1 bis 4 Kohlenstoffatomen,
mit der Maßgabe, daß die Reste Cycloalkyl, Cycloalkenyl oder Bicycloalkyl gegebenenfalls durch einen oder mehrere Alkylreste mit 1 bis 4 Kohlenstoffatomen substituiert sein können, und
Ph einen Rest Phenyl bedeutet, substituiert durch einen oder mehrere Reste Alkoxy mit 1 bis 4 Kohlenstoffatomen,
dadurch gekennzeichnet, daß man die Cyclisierung eines Produktes der allgemeinen Formel 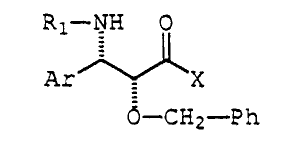 durchführt, in der Ar, R₁ und Ph wie oben definiert sind und X einen Rest L(+)-2,10-Camphorsultam der Formel 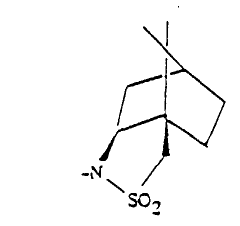 oder einen Rest -O-R darstellt, worin R einen Rest Alkyl mit 1 bis 4 Kohlenstoffatomen bedeutet, gegebenenfalls substituiert durch einen Rest Phenyl, wobei man in einem organischen Lösungsmittel und in Anwesenheit eines Oxidationsmittels bei einer Temperatur zwischen 0 °C und der Siedetemperatur der Reaktionsmischung arbeitet, gefolgt von der Hydrolyse oder der Verseifung des erhaltenen Produktes der allgemeinen Formel 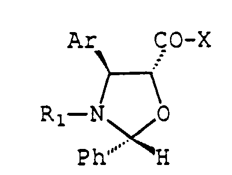 in der Ar, R₁, Ph und X wie oben definiert sind, unter bekannten Bedingungen.

2. Verfahren nach Anspruch 1, dadurch gekennzeichnet, daß das Lösungsmittel unter den Ethern, den Estern, den Ketonen, den Nitrilen, den aliphatischen, gegebenenfalls halogenierten Kohlenwasserstoffen und den aromatischen Kohlenwasserstoffen ausgewählt wird.

3. Verfahren nach einem der Ansprüche 1 oder 2, dadurch gekennzeichnet, daß das organische Lösungsmittel wasserfrei ist.

4. Verfahren nach Anspruch 1, dadurch gekennzeichnet, daß das Oxidationsmittel Dichlordicyanobenzochinon ist.

5. Verfahren nach einem der Ansprüche 1 bis 4, dadurch gekennzeichnet, daß X ein Rest L(+)-2,10-Camphorsultam der Formel (IV) 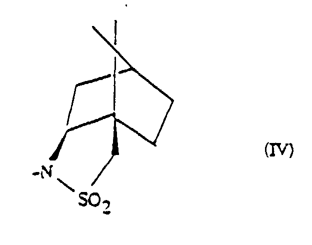 ist.

6. Verfahren zur Herstellung einer 1,3-Oxazolidin-5-carbonsäure wie in Anspruch 1 definiert, dadurch gekennzeichnet, daß man einen Ether der allgemeinen Formel
Ph-CH₂-O-R₃
in der Ph wie in Anspruch 1 definiert ist und R₃ einen Rest Alkyl mit 1 bis 4 Kohlenstoffatomen oder einen Rest Phenyl darstellt, mit einem Phenylisoserin-Derivat der allgemeinen Formel 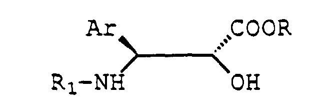 zur Reaktion bringt, in der Ar, R₁ und R wie in Anspruch 1 definiert sind, wobei man in einem organischen Lösungsmittel und in Anwesenheit eines Oxidationsmittels bei einer Temperatur zwischen 0 °C und der Siedetemperatur der Reaktionsmischung arbeitet, und man anschließend den erhaltenen Ester der allgemeinen Formel 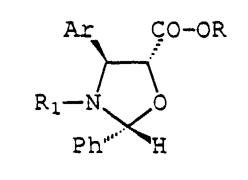 in der Ar, R₁, R und Ph wie in Anspruch 1 definiert sind, im basischen Medium nach bekannten Methoden verseift.

7. Verfahren nach Anspruch 6, dadurch gekennzeichnet, daß die Umsetzung des Ethers mit dem Phenylisoserin-Derivat in einem organischen Lösungsmittel und in Anwesenheit eines Oxidationsmittels bei einer Temperatur zwischen 0 °C und der Siedetemperatur der Reaktionsmischung durchgeführt wird.

8. Verfahren nach Anspruch 7, dadurch gekennzeichnet, daß das Lösungsmittel unter den Ethern, den Estern, den Ketonen, den Nitrilen, den aliphatischen, gegebenenfalls halogenierten Kohlenwasserstoffen und den aromatischen, gegebenenfalls halogenierten Kohlenwasserstoffen ausgewählt wird.

9. Verfahren nach Anspruch 8, dadurch gekennzeichnet, daß das organische Lösungsmittel wasserfrei ist.

10. Verfahren zur Herstellung von Taxan der allgemeinen Formel (XXI) 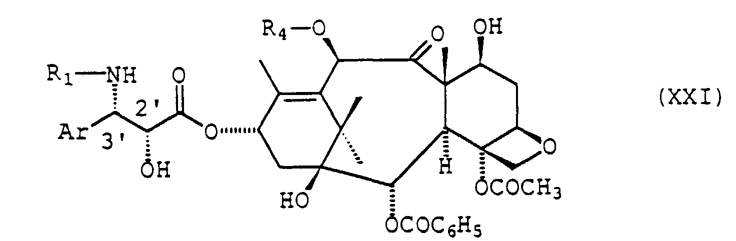 in der Ar und R₁ wie in Anspruch 1 definiert sind und R₄ ein Wasserstoffatom oder einen Rest Acetyl darstellt, dadurch gekennzeichnet, daß man durchführt:
- die Kondensation einer Säure der allgemeinen Formel (I), erhalten nach Anspruch 1, oder eines Derivates dieser Säure mit Baccatin III oder 10-Desacetyl-Baccatin III der allgemeinen Formel (XXII) 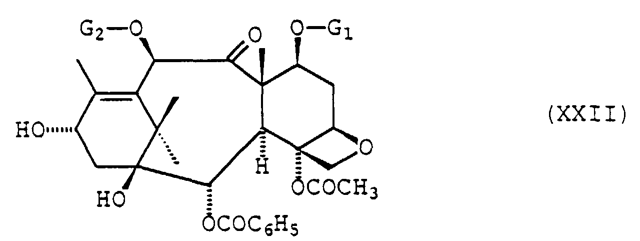 in der G₁ eine Schutzgruppe für die Hydroxyfunktion darstellt und G₂ einen Rest Acetyl oder eine Schutzgruppe für die Hydroxyfunktion bedeutet, um ein Produkt der allgemeinen Formel (XXIII) 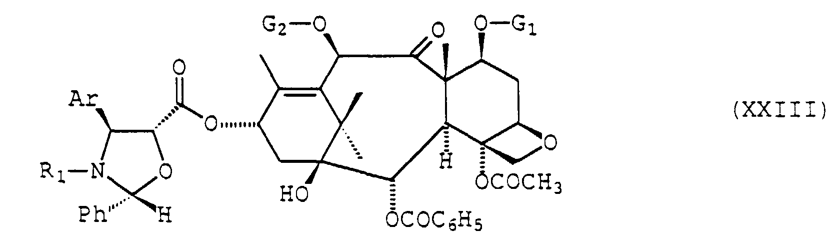 zu erhalten, in der Ar, R₁, Ph, G₁ und G₂ wie oben definiert sind,
- die Abspaltung der Schutzgruppe von der Seitenkette und gegebenenfalls von den durch G₁ und G₂ geschützten Hydroxyfunktionen, um ein Produkt der allgemeinen Formel (XXIV) 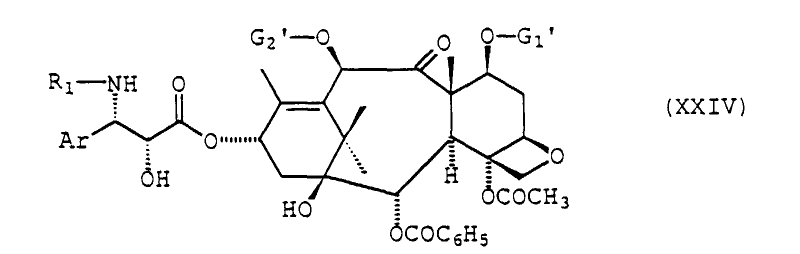 zu erhalten, in der Ar und R₁ wie oben definiert sind, G₁' ein Wasserstoffatom oder eine Schutzgruppe für die Hydroxyfunktion darstellt und G₂' ein Wasserstoffatom oder einen Rest Acetyl oder eine Schutzgruppe für die Hydroxyfunktion bedeutet,
- anschließend gegebenenfalls der Austausch der Schutzgruppen G₁' und gegebenenfalls G₂' bei dem Produkt der allgemeinen Formel (XXIV) durch Wasserstoffatome, um ein Produkt der allgemeinen Formel (XXI) zu erhalten.

11. Verfahren nach Anspruch 10, dadurch gekennzeichnet, daß die Kondensation in Anwesenheit eines Kondensationsmittels durchgeführt wird, ausgewählt unter den Carbodiimiden oder den reaktiven Carbonaten, und eines Aktivierungsmittels, ausgewählt unter den Aminopyridinen.

12. Verfahren nach Anspruch 10, dadurch gekennzeichnet, daß die Kondensation in einem organischen Lösungsmittel durchgeführt wird, ausgewählt unter den Ethern, den Ketonen, den Estern, den Nitrilen, den aliphatischen, gegebenenfalls halogenierten Kohlenwasserstoffen und den aromatischen Kohlenwasserstoffen.

13. Verfahren nach Anspruch 10, dadurch gekennzeichnet, daß das Säurederivat der Formel (I) ein Anhydrid der allgemeinen Formel (XXV) 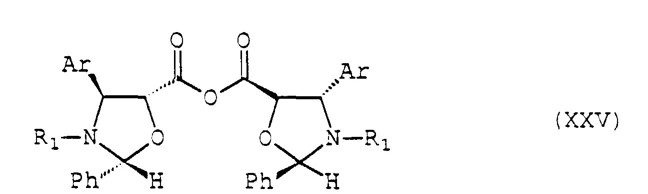 ist, in der Ar, R₁ und Ph wie in Anspruch 1 definiert sind.

14. Verfahren nach Anspruch 10, dadurch gekennzeichnet, daß das Säurederivat der Formel (I) ein Halogenid oder ein gemischtes Anhydrid der Formel (XXVI) 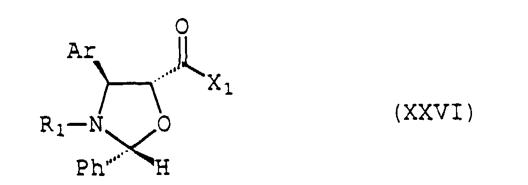 ist, in der Ar, R₁ und Ph wie in Anspruch 1 definiert sind und X₁ ein Halogenatom oder einen Rest Acyloxy mit 1 bis 5 Kohlenstoffatomen oder Aryloxy darstellt, worin der Arylteil ein Rest Phenyl ist, gegebenenfalls substituiert durch 1 bis 5 gleiche oder verschiedene Reste, ausgewählt unter den Halogenatomen und den Resten Nitro, Methyl oder Methoxy.

15. Verfahren nach Anspruch 10, dadurch gekennzeichnet, daß die Abspaltung der Schutzgruppe von der Seitenkette in Anwesenheit einer Mineralsäure oder organischen Säure durchgeführt wird.

16. Verfahren nach Anspruch 15, dadurch gekennzeichnet, daß die Abspaltung der Schutzgruppe in einem organischen Lösungsmittel durchgeführt wird, ausgewählt unter den Alkoholen, den Ethern, den Estern, den aliphatischen Kohlenwasserstoffen, den halogenierten aliphatischen Kohlenwasserstoffen, den aromatischen Kohlenwasserstoffen und den Nitrilen.

17. Verfahren nach Anspruch 10, dadurch gekennzeichnet, daß die Abspaltung der Schutzgruppe unter oxidierenden Bedingungen und unter Verwendung von Ammoniumnitrat und Cer(IV) -nitrat oder 2,3-Dichlor-5,6-dicyano-1,4-benzochinon durchgeführt wird.

18. Verfahren nach Anspruch 10, dadurch gekennzeichnet, daß die Schutzgruppen G₁ und G₂ Reste 2,2,2-Trichlor-ethoxycarbonyl, 2-(2-Trichlormethyl-propoxy)-carbonyl, Benzyl, 4-Alkoxybenzyl oder 2,4-Dialkoxybenzyl sind, worin die Reste Alkoxy 1 bis 4 Kohlenstoffatome enthalten, oder auch Reste Trialkylsilyl, Dialkylarylsilyl, Alkyldiarylsilyl oder Triarylsilyl darstellen, worin die Alkylteile 1 bis 4 Kohlenstoffatome enthalten und die Arylteile Phenylreste sind.

19. Verfahren nach Anspruch 10, dadurch gekennzeichnet, daß die Abspaltung der Schutzgruppen G₁ und gegebenenfalls G₂, die einen Rest 2,2,2-Trichlor-ethoxycarbonyl oder 2-(2-Trichlormethyl-propoxy)-carbonyl darstellen, mit Hilfe einer Behandlung durch Zink, gegebenenfalls assoziiert mit Kupfer, durchgeführt wird.

## Claims

1. Process for the preparation of a 1,3-oxazolidine-5-carboxylic acid of general formula: 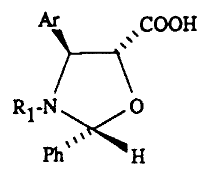 in which
Ar represents a phenyl or α- or β-naphthyl radical optionally substituted by one or a number of atoms or radicals, which are identical or different, chosen from halogen atoms (fluorine, chlorine, bromine, iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano and trifluoromethyl radicals, it being understood that the alkyl radicals and the alkyl portions of the other radicals contain 1 to 4 carbon atoms, that the alkenyl and alkynyl radicals contain 3 to 8 carbon atoms and the aryl radicals are phenyl or α- or β-naphthyl radicals,
R₁ represents a benzoyl radical or a radical R₂-O-CO- in which R₂ represents
- a straight or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 11 carbon atoms, these radicals optionally being substituted by one or a number of substituents chosen from halogen atoms and hydroxyl, alkyloxy containing 1 to 4 carbon atoms, dialkylamino, in which each alkyl part contains 1 to 4 carbon atoms, piperidino, morpholino, 1-piperazinyl (optionally substituted in the 4-position by an alkyl radical containing 1 to 4 carbon atoms or by a phenylalkyl radical in which the alkyl part contains 1 to 4 carbon atoms), cycloalkyl containing 3 to 6 carbon atoms, cycloalkenyl containing 4 to 6 carbon atoms, phenyl, cyano, carboxyl or alkyloxycarbonyl, in which the alkyl part contains 1 to 4 carbon atoms, radicals,
- or a phenyl radical optionally substituted by one or a number of atoms or radicals chosen from alkyl radicals containing 1 to 4 carbon atoms or alkyloxy radicals containing 1 to 4 carbon atoms,
- or a saturated or unsaturated nitrogenous heterocyclyl radical containing 5 or 6 members and optionally substituted by one or a number of alkyl radicals containing 1 to 4 carbon atoms,
it being understood that the cycloalkyl, cycloalkenyl or bicycloalkyl radicals can be optionally substituted by one or a number of alkyl radicals containing 1 to 4 carbon atoms, and
Ph represents a phenyl radical substituted by one or a number of alkoxy radicals containing 1 to 4 carbon atoms,
characterized in that a product of general formula: 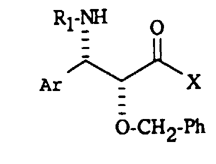 in which Ar, R₁ and Ph are defined as above and X represents an L-(+)-2,10-camphorsultam residue of formula 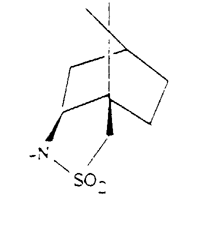 or a residue -O-R in which R represents an alkyl radical containing 1 to 4 carbon atoms optionally substituted by a phenyl radical, is cyclized, the cyclization being carried out in an organic solvent in the presence of an oxidizing agent at a temperature between 0°C and the boiling temperature of the reaction mixture, followed by the hydrolysis or saponification, under known conditions, of the product obtained of general formula: 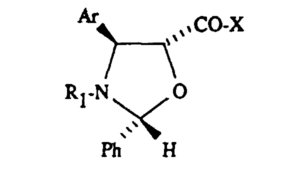 in which Ar, R₁, Ph and X are defined as above.

2. Process according to claim 1, characterized in that the solvent is chosen from ethers, esters, ketones, nitriles, optionally halogenated aliphatic hydrocarbons, and aromatic hydrocarbons.

3. Process according to either of claims 1 and 2, characterized in that the organic solvent is anhydrous.

4. Process according to claim 1, characterized in that the oxidizing agent is dichlorodicyanobenzoquinone.

5. Process according to one of claims 1 to 4, characterized in that X is an L-(+)-2,10-camphorsultam residue of formula:

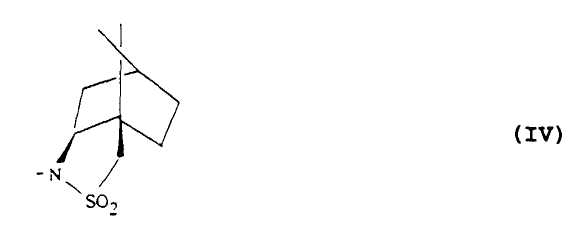

6. Process for the preparation of a 1,3-oxazolidine-5-carboxylic acid as defined in claim 1, characterized in that an ether of general formula:
Ph-CH₂-O-R₃
in which Ph is defined as in claim 1 and R₃ represents an alkyl radical containing 1 to 4 carbon atoms or a phenyl radical, is reacted with a phenylisoserine derivative of general formula 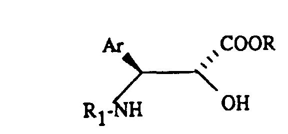 in which Ar, R₁ and R are defined as in claim 1, the reaction being carried out in an organic solvent in the presence of an oxidizing agent at a temperature between 0°C and the boiling temperature of the reaction mixture, and then the ester obtained of general formula: 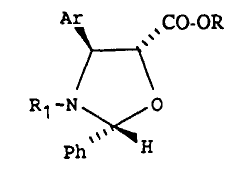 in which Ar, R₁, R and Ph are defined as in claim 1, is saponified in basic medium according to known methods.

7. Process according to claim 6, characterized in that the reaction of the ether with the phenylisoserine derivative is carried out in an organic solvent in the presence of an oxidizing agent at a temperature between 0°C and the boiling temperature of the reaction mixture.

8. Process according to claim 7, characterized in that the solvent is chosen from ethers, esters, ketones, nitriles, optionally halogenated aliphatic hydrocarbons and optionally halogenated aromatic hydrocarbons.

9. Process according to claim 8, characterized in that the organic solvent is anhydrous.

10. Process for the preparation of taxane of general formula 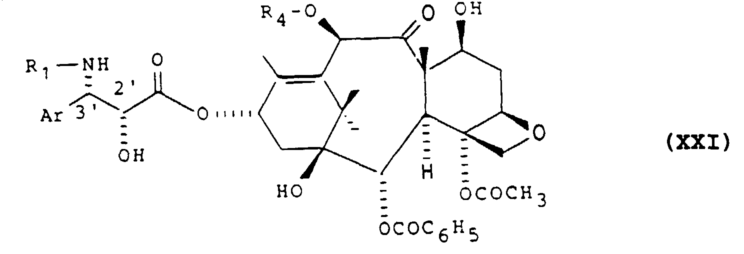 in which Ar and R₁ are defined as in claim 1 and R₄ represents a hydrogen atom or an acetyl radical,
characterized in that
- an acid of general formula (I) obtained according to claim 1, or a derivative of this acid, is condensed with baccatin III or 10-deacetylbaccatin III of general formula: 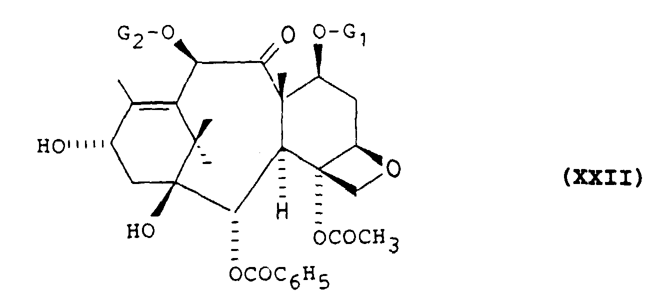 in which G₁ represents a protective group of the hydroxyl functional group and G₂ represents an acetyl radical or a protective group of the hydroxyl functional group, to obtain a product of general formula: 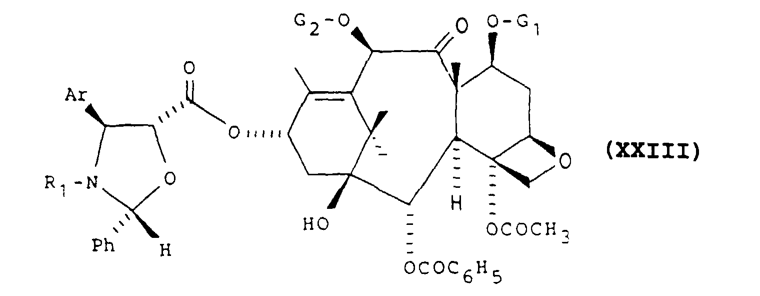 in which Ar, R₁, Ph, G₁ and G₂ are defined as above,
- the side chain and optionally the hydroxyl functional groups protected by G₁ and G₂ are deprotected to obtain a product of general formula: 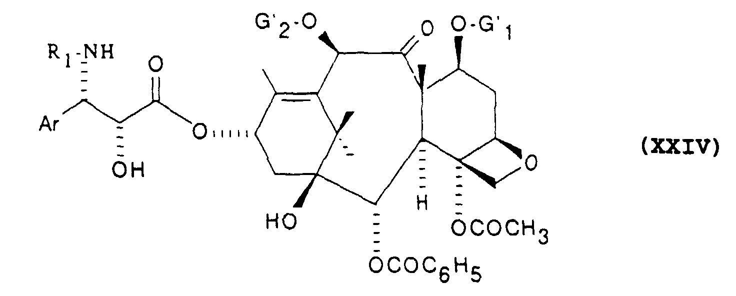 in which Ar and R₁ are defined as above, G'₁ represents a hydrogen atom or a protective group of the hydroxyl functional group and G'₂ represents a hydrogen atom or an acetyl radical or a protective group of the hydroxyl functional group,
- the G'₁ and optionally G'₂ protective groups of the product of general formula (XXIV) are then optionally replaced by hydrogen atoms to obtain a product of general formula (XXI).

11. Process according to claim 10, characterized in that the condensation is carried out in the presence of a condensation agent chosen from carbodiimides or reactive carbonates and of an activating agent chosen from aminopyridines.

12. Process according to claim 10, characterized in that the condensation is carried out in an organic solvent chosen from ethers, ketones, esters, nitriles, optionally halogenated aliphatic hydrocarbons or aromatic hydrocarbons.

13. Process according to claim 10, characterized in that the derivative of the acid of formula (I) is an anhydride of general formula 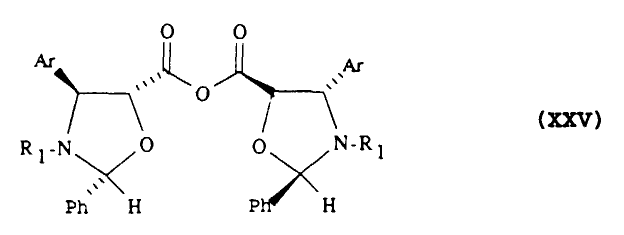 in which Ar, R₁ and Ph are defined as in claim 1.

14. Process according to claim 10, characterized in that the derivative of the acid of formula (I) is a halide or a mixed anhydride of formula 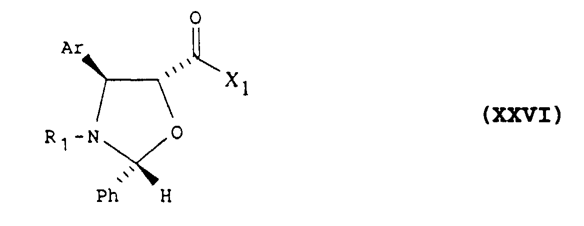 in which Ar, R₁ and Ph are defined as in claim 1 and X₁ represents a halogen atom or an acyloxy radical containing 1 to 5 carbon atoms or an aroyloxy radical in which the aryl part is a phenyl radical optionally substituted by 1 to 5 radicals, which are identical or different, chosen from halogen atoms or nitro, methyl or methoxy radicals.

15. Process according to claim 10, characterized in that the deprotection of the side chain is carried out in the presence of an inorganic or organic acid.

16. Process according to claim 15, characterized in that the deprotection is carried out in an organic solvent chosen from alcohols, ethers, esters, aliphatic hydrocarbons, halogenated aliphatic hydrocarbons, aromatic hydrocarbons and nitriles.

17. Process according to claim 10, characterized in that the deprotection is carried out under oxidizing conditions by using cerium(IV) ammonium nitrate or 2,3-dichloro-5,6-dicyano-1,4-benzoquinone.

18. Process according to claim 10, characterized in that the protective groups G₁ and G₂ are 2,2,2-trichloroethoxycarbonyl, 2-(2-(trichloromethyl)propoxy)carbonyl, benzyl, 4-alkoxybenzyl or 2,4-dialkoxybenzyl radicals in which the alkoxy radicals contain 1 to 4 carbon atoms or trialkylsilyl, dialkylarylsilyl, alkyldiarylsilyl or triarylsilyl radicals in which the alkyl parts contain 1 to 4 carbon atoms and the aryl parts are phenyl radicals.

19. Process according to claim 10, characterized in that the deprotection of the G₁ and optionally G₂ protective groups representing a 2,2,2-trichloroethoxycarbonyl or 2-(2-(trichloromethyl)propoxy)carbonyl radical is carried out with zinc, optionally in combination with copper.
